# FASCICULE DE BREVET EUROPEEN

(11) **EP 0 718 005 B1**
(45) Date de publication et mention de la délivrance du brevet: **12.09.2001**
(21) Numéro de dépôt: 94402965.1
(22) Date de dépôt: 21.12.1994
(51) Int. Cl.: A61M 39/28, A61M 5/40

(54) **Dispositif pour perfusion**
Infusionsvorrichtung
Infusion device

(43) Date de publication de la demande: 26.06.1996
(73) Titulaire: David, François, F-75008 Paris (FR); David, Jean, F-91140 Villebon/Yvette (FR)
(72) Inventeur: David, François, F-75008 Paris (FR); David, Jean, F-91140 Villebon/Yvette (FR)
(74) Mandataire: Flavenot, Bernard

(56) Documents cités:
- WO-A-87/05225
- FR-A- 2 273 264
- FR-A- 2 321 904
- GB-A- 2 079 410
- US-A- 2 653 787

## Description

La présente invention concerne les dispositifs pour perfusions, ci-après dénommés "perfuseurs", trouvant une application dans le domaine médical pour perfuser des malades.

Ces dispositifs comportent une chambre à deux premier et second orifices, des moyens pour relier le premier orifice à une source de fluide médical, une tubulure en un matériau relativement souple, et des seconds moyens pour relier le second orifice et la tubulure, la tubulure comportant généralement à son autre extrémité un cathéter, une aiguille ou analogue pour qu'elle puisse être reliée, par exemple, à une veine du patient.

Les perfuseurs du type défini ci-dessus qui sont actuellement sur le marché ne donnent pas toujours satisfaction, car ils ne permettent pas d'assurer toutes les fonctions qui peuvent être demandées dans les unités de soins des hôpitaux, cliniques et autres établissements. Ces fonctions sont par exemple: leur positionnement à une altitude supérieure à celle où se trouve le patient, la possibilité d'être déplacés sur une certaine distance en même temps que le malade auquel il est relié, la régulation du débit du fluide qu'il contient, et l'émission d'une alarme lorsque, par exemple, la chambre contenant le fluide à perfuser est presque vide.

Le document FR-A-2 273 264 décrit un perfuseur de liquide selon le préambule de la revendication 1.

La présente invention a ainsi pour but de réaliser un perfuseur qui ait une structure compacte relativement simple à réaliser, qui permette d'assurer les fonctions mentionnées ci-dessus et qui soit d'une utilisation facile, notamment pour le personnel soignant.

Plus précisément, la présente invention telle que décrite dans la rev. 1, a pour objet un perfuseur de fluide pour traitement médical, comportant une chambre comprenant au moins deux premier et second orifices, des premiers moyens pour relier le premier orifice à une source dudit fluide médical, une tubulure en un matériau relativement souple, et des seconds moyens pour relier le second orifice à ladite tubulure,
- un support constitué d'un montant et d'une base, ladite base comportant deux première et seconde extrémités et étant solidaire dudit montant par sa première extrémité de façon que ledit montant et ladite base forment sensiblement un "L",
- des moyens de fixation de ladite chambre par rapport au montant dudit support, et
- des moyens commandables pour aplatir ladite tubulure contre ladite base pour faire varier de façon déterminée la valeur de sa section.

D'autres caractéristiques et avantages de la présente invention apparaîtront au cours de la description suivante donnée en regard des dessins annexés à titre illustratif, mais nullement limitatif, dans lesquels:
La figure 1 représente une vue en coupe d'un mode de réalisation d'un perfuseur selon l'invention, cette coupe étant référencée I-I sur la figure 2, et
La figure 2 représente une vue en coupe du mode de réalisation selon la figure 1, cette coupe étant référencée II-II sur la figure 1.

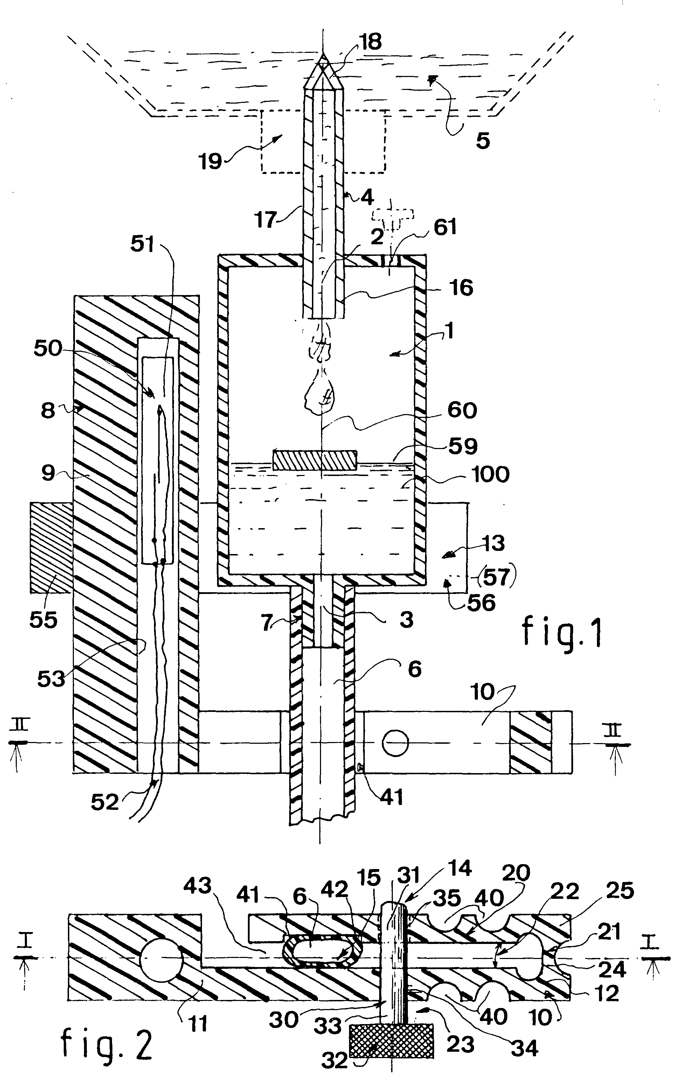

Les figures 1 et 2 représentent un même mode de réalisation d'un perfuseur de fluide 100 pour traitement médical. Ce perfuseur comporte une chambre 1 généralement de forme cylindrique et avantageusement en un matériau transparent permettant l'observation du fluide qu'elle contient et permettant de suivre l'évolution des conditions dans lesquelles s'opère la perfusion. Cette chambre comprend au moins deux premier 2 et second 3 orifices, des premiers moyens 4 pour relier le premier orifice 2 à une source 5 du fluide médical, une tubulure 6 en un matériau relativement souple et élastique, et des seconds moyens 7 pour relier le second orifice 3 à l'une des extrémités de la tubulure.

La source de fluide 5 est généralement constituée d'une poche ou analogue qui contient une certaine quantité de fluide nécessaire pour assurer une perfusion pendant un temps déterminé suffisamment long. Cette poche comporte en outre un bouchon 19 en une matière lui permettant d'être perforé. Dans ce cas, les premiers moyens 4 pour relier la chambre 1 à la source de fluide 5 peuvent être constitués par un conduit 17 relativement rigide terminé par une pointe ouverte 18 apte à perforer le bouchon 19 pour pénétrer dans la poche, l'autre extrémité 16 de ce conduit 17 plongeant dans la chambre 1 pour donner un écoulement du fluide de type goutte-à-goutte.

Les seconds moyens 7 pour relier le second orifice 3 à l'une des extrémités de la tubulure 6 sont généralement simplement constitués par un emmanchement en force sur un manchon bordant l'orifice 3, comme illustré sur la figure 1.

Selon une caractéristique de la présente invention, le perfuseur comporte un support 8 constitué d'un montant 9 et d'une base 10, la base comportant deux première 11 et seconde 12 extrémités et étant solidaire du montant 9 par sa première extrémité 11 de façon que le montant et la base forment sensiblement un "L", comme illustré sur la figure 1. L'angle entre le montant et la base est, sur l'exemple illustré, de quatre-vingt-dix degrés, mais cette valeur, bien que certainement avantageuse, n'est pas obligatoirement nécessaire.

Le montant présente avantageusement une forme qui lui permet d'être facilement associé, par exemple, à un pied comportant des roulettes comme ceux que l'on utilise dans les établissements médicaux pour le transport des perfuseurs lorsqu'ils sont reliés à un malade.

Le perfuseur comporte aussi des moyens de fixation 13 de la chambre 1 par rapport au support 8. Ces moyens sont plus particulièrement adaptés pour que l'axe longitudinal 60 de la chambre soit parallèle au montant 9 et avantageusement sensiblement contenu dans le plan de symétrie du montant 9 et de la base 10, et pour que le second orifice 3 de la chambre soit sensiblement au-dessus de cette base 10, la tubulure 6 passant de façon sensiblement tangentielle à la base 10. Ces moyens de fixation 13 sont de façon préférentielle constitués par une pince 55 pinçant simultanément, entre deux mâchoires 56, 57, la chambre 1 et le montant 9, cette pince 55 pouvant être constituée en un matériau qui présente une certaine élasticité pour permettre ce pincement.

Le perfuseur comporte aussi des moyens commandables 14 pour aplatir la tubulure 6 contre la base 10 et faire varier de façon déterminée la valeur de sa section 15 afin de réguler le débit du fluide qui s'écoule dans cette tubulure, et donc le débit de la perfusion.

Selon un mode de réalisation préférentiel, les moyens commandables 14 pour aplatir la tubulure 6 contre la base 10 comportent une patte 20 et des moyens 21 pour monter la patte rotative sur la base, la tubulure 6 passant alors entre la patte 20 et la base 10.

Des moyens pour exercer une force élastique entre la base 10 et la patte 20 pour tendre à faire augmenter la valeur de l'angle 22 que fait la patte 20 avec la base 10 sont prévus en association avec des moyens 23 pour commander de façon déterminée, à l'encontre de la force élastique, une diminution de la valeur de cet angle 22.

Il est bien précisé que, par "augmentation de la valeur de l'angle 22" on entend la variation de la valeur de cet angle quand la base 10 et la patte 20 entre lesquelles passe la tubulure s'éloignent l'une de l'autre pour augmenter la section 15 de la tubulure 6 lorsqu'elle a été aplatie et que, par "diminution de la valeur de cet angle 22", on entend la variation de la valeur de cet angle quand la base 10 et la patte 20 se rapprochent l'une de l'autre pour diminuer la section 15 de la tubulure 6.

Les moyens 21 pour monter la patte 20 rotative sur la base 10 sont avantageusement constitués par une paroi déformable 24 solidaire d'une extrémité 25 de la patte 20 et de la seconde extrémité 12 de la base 10 de façon que la base et la patte soient, normalement, sensiblement parallèles et en regard l'une de l'autre en étant espacées d'une distance sensiblement égale à l'épaisseur de la tubulure 6 lorsqu'elle n'est pas aplatie.

Cette structure est avantageuse car elle permet de réaliser le montant 9, la base 10, la paroi déformable 24 et la patte 20 en une pièce unitaire, la base, la paroi déformable et la patte formant sensiblement un "U" et les moyens pour exercer la force élastique entre la base et la patte étant dans ce cas constitués par le fait que la pièce unitaire est réalisée dans un matériau présentant une certaine résilience.

Les moyens 23 pour commander la variation de la valeur de l'angle 22 comme défini ci-avant sont constitués par une vis 30 comportant une tige filetée 31 et une tête 32 solidarisée à une extrémité 33 de la tige filetée, la tige 31 traversant l'un 10 des deux éléments, patte 20 et base 10, par une percée 34 d'un diamètre au moins égal au diamètre de la tige 31 et coopérant avec une percée 35 réalisée dans l'autre élément 20 et taraudée au même pas de vissage que celui de la tige filetée.

De cette façon, en tournant dans un sens ou dans l'autre la tête de la vis 30, on rapproche ou on éloigne la patte 20 et la base 10 en écrasant la tubulure 6 ou en lui permettant de reprendre sa forme initiale. Pour cela, comme mentionné ci-avant, la tubulure 6 est réalisée dans un matériau souple mais présentant une certaine élasticité. Certains matériaux plastiques bien connus des hommes du métier présentent ces caractéristiques.

L'ensemble des quatre éléments définis ci-dessus est, dans le mode de réalisation décrit, réalisé en une pièce unitaire dans un matériau présentant une certaine résilience. Cependant, pour que, seules, la patte 20 et la base 10 soient facilement élastiquement déformables, ces deux derniers éléments comportent des découpes longitudinales 40 dont les axes sont sensiblement perpendiculaires au plan de rotation de la patte 20 par rapport à la base 10.

Comme mentionné ci-avant, la tubulure 6 est pincée entre la patte 20 et la base 10. Pour qu'elle reste au même endroit et ne glisse pas, soit vers le fond du "U" soit hors de celui-ci, le perfuseur comporte au moins une gorge de réception 41 de la tubulure 6 réalisée dans l'un 20 des deux éléments, patte 20 et base 10, de façon qu'elle soit perpendiculaire au plan de rotation de la patte par rapport à la base et ouverte en 42 dans l'espace 43 défini entre ces deux éléments 10, 20.

Dans un mode de réalisation avantageux, la profondeur de cette gorge 41 est au maximum égale au double de l'épaisseur de la paroi de la tubulure 6 de façon que la tubulure puisse être complètement écrasée et donc totalement obturée lorsque la base et la patte sont au contact l'une de l'autre.

Comme mentionné ci-avant, le perfuseur selon l'invention permet de détecter l'instant où le niveau du fluide 100 dans la chambre 1 descend en dessous d'un niveau de référence prédéterminé.

Dans ce but, le perfuseur comporte des moyens de détection 50 du niveau 59 du fluide 100 lorsqu'il arrive au niveau de référence. Ces moyens 50 comportent un capteur 51 apte à délivrer un signal en sortie 52 lorsque le niveau 59 du fluide dans la chambre 1 atteint le niveau de référence. Le capteur 51 est disposé dans un logement 53 réalisé dans le montant 9 et peut être de différents types, par exemple une fibre optique ou un interrupteur à lame souple en association avec un aimant monté dans un flotteur disposé dans la chambre 1, le flotteur suivant le niveau 59 du fluide et fermant l'interrupteur à lame souple lorsqu'il arrive sur le niveau de référence. L'interrupteur ferme alors un circuit, par exemple électrique, dans lequel est montée, par exemple, une alarme sonore, optique, etc.

Il est en outre préférable, pour que le perfuseur puisse fonctionner quel que soit le mode de réalisation de la poche constituant la source de fluide 5, que la chambre 1 comporte, par exemple sensiblement au niveau du premier orifice 2, un évent 61 qui puisse être fermé ou ouvert, par exemple à l'aide un bouchon, selon que la poche soit, ou non, munie d'une ouverture permettant la mise à l'air libre du fluide qu'elle contient, de façon que ce fluide puisse s'écouler parfaitement et totalement.

A la description ci-dessus, on constate que la structure du perfuseur selon l'invention est parfaitement compacte et permet d'atteindre tous les buts qui ont été mentionnés au préambule.

## Revendications

1. Perfuseur de fluide (100) pour traitement médical, comportant une chambre (1) comprenant au moins deux premier (2) et second (3) orifices, des premiers moyens (4) pour relier le premier orifice (2) à une source (5) dudit fluide médical, une tubulure (6) en un matériau relativement souple, des seconds moyens (7) pour relier le second orifice (3) à ladite tubulure et des moyens commandables (14) pour aplatir ladite tubulure (6) pour faire varier de façon déterminée la valeur de sa section (15),
un support (8) comportant une base (10), ladite base comportant deux première (11) et seconde (12) extrémités
lesdits moyens commandables (14) étant aptes à aplatir ladite tubulure (6) contre ladite base (10), **caractérisé par** le fait qu'il comporte en outre:
- des moyens de fixation (13) de ladite chambre (1) par rapport montant (9) dudit support (8),
ledit support (8) étant constitué d'un seul montant (9),
ladite base (10) étant solidaire dudit montant par sa première extrémité (11) de façon que ledit montant et ladite base forment sensiblement un "L".

2. Perfuseur selon la revendication 1, **caractérisé par** le fait que les moyens commandables (14) pour aplatir ladite tubulure (6) contre ladite base (10) comportent une patte (20), des moyens (21) pour monter ladite patte rotative sur ladite base, des moyens pour exercer une force élastique entre ladite base et ladite patte pour tendre à augmenter la valeur de l'angle (22) entre ladite patte (20) et ladite base (10), et des moyens (23) pour commander de façon déterminée, à l'encontre de ladite force élastique, une diminution de la valeur dudit angle (22).

3. Perfuseur selon la revendication 2, **caractérisé par** le fait que les moyens (21) pour monter ladite patte (20) rotative sur ladite base (10) sont constitués par une paroi déformable (24) solidaire d'une extrémité (25) de ladite patte (20) et de la seconde extrémité (12) de ladite base (10) de façon que ladite base et ladite patte soient, normalement, sensiblement parallèles et en regard l'une de l'autre en étant espacées d'une distance sensiblement égale à l'épaisseur de ladite tubulure lorsqu'elle n'est pas aplatie.

4. Perfuseur selon la revendication 3, **caractérisé par** le fait que lesdites patte (20), paroi déformable (24) et base (10) forment une pièce unitaire en forme sensiblement de "U", et que les moyens pour exercer une force élastique entre ladite base et ladite patte sont constitués par le fait que ladite pièce unitaire est réalisée dans un matériau présentant une certaine résilience.

5. Perfuseur selon l'une des revendications 2 à 4, **caractérisé par** le fait que les moyens (23) pour commander la variation de la valeur dudit angle (22) entre ladite patte (20) et ladite base (10) sont constitués par une vis (30) comportant une tige filetée (31) et une tête (32) solidarisée à une extrémité (33) de ladite tige filetée (31), ladite tige (31) traversant l'un (10) des deux éléments, patte (20) et base (10), par une percée (34) d'un diamètre au moins égal au diamètre de ladite tige (31) et coopérant avec une percée (35) réalisée dans l'autre élément (20) et taraudée au même pas que celui de ladite tige filetée .

6. Perfuseur selon l'une des revendications 4 et 5, **caractérisé par** le fait que ladite pièce unitaire comporte des découpes longitudinales (40) dont les axes sont sensiblement perpendiculaires au plan de rotation de ladite patte (20) par rapport à ladite base (10).

7. Perfuseur selon l'une des revendications 2 à 6, **caractérisé par** le fait qu'il comporte au moins une gorge de réception (41) de ladite tubulure (6), ladite gorge (41) étant réalisée dans l'un (20) des deux éléments, patte (20) et base (10), de façon qu'elle soit perpendiculaire au plan de rotation de la patte par rapport à la base et ouverte (42) dans l'espace (43) défini entre ces deux dits éléments (10, 20).

8. Perfuseur selon l'une des revendications 1 à 7, **caractérisé par** le fait qu'il comporte des moyens de détection (50) du niveau (59) dudit fluide (100) dans ladite chambre (1) lorsqu'il atteint un niveau de référence.

9. Perfuseur selon la revendication 8, **caractérisé par** le fait que les moyens de détection (50) comportent au moins un capteur (51) apte à délivrer un signal en sortie (52) lorsque ledit niveau du fluide dans ladite chambre (1) atteint le niveau de référence, ledit capteur (51) étant disposé dans un logement (53) réalisé dans ledit montant (9).

10. Perfuseur selon l'une des revendications 1 à 9, **caractérisé par** le fait que les moyens de maintien (13) de ladite chambre (1) par rapport audit support (8) sont constitués par une pince (55) pinçant simultanément, entre deux mâchoires (56, 57), ladite chambre (1) et ledit montant (9).

## Patentansprüche

1. Fluid-Perfusionseinrichtung (100) zur medizinischen Behandlung, mit einer Kammer (1), die wenigstens zwei erste (2) und zweite (3) Öffnungen, erste Mittel (4) zum Verbinden der ersten Öffnung (2) mit einer Quelle (5) des medizinischen Fluids, eine Rohrleitung (6) aus einem verhältnismäßig weichen Werkstoff, zweite Mittel (7) zum Verbinden der zweiten Öffnung (3) mit der Rohrleitung sowie steuerbare Mittel (14) zum Quetschen der Rohrleitung (6), um den Wert ihres Querschnitts (15) in bestimmter Weise zu verändern, enthält,
einem Träger (8), der eine Basis (10) enthält, die zwei erste (11) und zweite (12) Enden besitzen,
wobei die steuerbaren Mittel (14) die Rohrleitung (6) gegen die Basis (10) quetschen können,
**dadurch gekennzeichnet, daß** sie außerdem umfaßt:
- Mittel (13) zum Befestigen der Kammer (1) in bezug auf eine Stütze (9) des Trägers (8), wobei der Träger aus einer einzigen Stütze (9) gebildet ist, wobei die Basis (10) mit der Stütze über ihr erstes Ende (11) in der Weise fest verbunden ist, daß die Stütze und die Basis im wesentlichen ein "L" bilden.

2. Perfusionseinrichtung nach Anspruch 1, **dadurch gekennzeichnet, daß** die steuerbaren Mittel (14) zum Quetschen der Rohrleitung (6) gegen die Basis (10) einen Ansatz (20), Mittel (21), die den Ansatz an der Basis drehbar festhalten, Mittel zum Ausüben einer elastischen Kraft zwischen der Basis und dem Ansatz, um zu versuchen, den Wert des Winkels (22) zwischen dem Ansatz (20) und der Basis (10) zu erhöhen, und Mittel (23), die in bestimmter Weise entgegen der elastischen Kraft eine Verringerung des Wertes des Winkels (22) steuern, umfassen.

3. Perfusionseinrichtung nach Anspruch 2, **dadurch gekennzeichnet, daß** die Mittel (21) zum drehbaren Anbringen des Ansatzes (20) an der Basis (10) durch eine verformbare Wand (24) gebildet sind, die mit einem Ende (25) des Ansatzes (22) und mit dem zweiten Ende (12) der Basis (10) in der Weise fest verbunden ist, daß die Basis und der Ansatz normalerweise im wesentlichen parallel sind und sich einander gegenüber befinden und dabei um eine Strecke beabstandet sind, die im wesentlichen gleich der Dicke der Rohrleitung ist, wenn sie nicht gequetscht ist.

4. Perfusionseinrichtung nach Anspruch 3, **dadurch gekennzeichnet, daß** der Ansatz (20), die verformbare Wand (24) und die Basis (10) ein einziges Teil, das im wesentlichen die Form eines "U" besitzt, bilden und daß die Mittel zum Ausüben einer elastischen Kraft zwischen der Basis und dem Ansatz dadurch gebildet sind, daß das einzige Teil aus einem Werkstoff hergestellt ist, der eine bestimmte Elastizität aufweist.

5. Perfusionseinrichtung nach einem der Ansprüche 2 bis 4, **dadurch gekennzeichnet, daß** die Mittel (23) zum Steuern der Änderung des Wertes des Winkels (22) zwischen dem Ansatz (20) und der Basis (10) durch eine Schraube (30) gebildet sind, die einen Gewindestift (31) und einen mit einem Ende (33) des Gewindestifts (31) fest verbundenen Kopf (32) umfaßt, wobei der Stift (31) durch eines der beiden Elemente, Ansatz (20) und Basis (10), durch ein Loch (34) mit einem Durchmesser, der wenigstens gleich dem Durchmesser des Stifts (31) ist, verläuft und mit einem Loch (35) zusammenwirkt, das im anderen Element (20) ausgebildet und mit einem Gewinde mit der gleichen Steigung wie jene des Gewindestifts versehen ist.

6. Perfusionseinrichtung nach einem der Ansprüche 4 und 5, **dadurch gekennzeichnet, daß** das einzige Teil longitudinale Einschnitte (40) aufweist, deren Achsen zur Drehebene des Ansatzes (20) in bezug auf die Basis (10) im wesentlichen senkrecht sind.

7. Perfusionseinrichtung nach einem der Ansprüche 2 bis 6, **dadurch gekennzeichnet, daß** sie wenigstens eine Äufnahmenut (41) der Rohrleitung (6) umfaßt, wobei die Nut (41) in einem (20) der beiden Elemente, Ansatz (20) und Basis (10), in der Weise ausgebildet ist, daß sie zur Drehebene des Ansatzes in bezug auf die Basis senkrecht ist und in den Raum (43), der zwischen diesen beiden Elementen (10, 20) definiert ist, mündet.

8. Perfusionseinrichtung nach einem der Ansprüche 1 bis 7, **dadurch gekennzeichnet, daß** sie Mittel (50) umfaßt, die den Pegel (59) des Fluids (100) in der Kammer (1) erfassen, wenn dieses einen Referenzpegel erreicht,.

9. Perfusionseinrichtung nach Anspruch 8, **dadurch gekennzeichnet, daß** die Erfassungsmittel (50) wenigstens einen Sensor (51) umfassen, der ein Ausgangssignal (52) liefern kann, wenn der Fluidpegel in der Kammer (1) den Referenzpegel erreicht, wobei der Sensor (51) in einem Aufnahmesitz (53) angeordnet ist, der in der Stütze (9) ausgebildet ist.

10. Perfusionseinrichtung nach einem der Ansprüche 1 bis 9, **dadurch gekennzeichnet, daß** die Mittel (13) zum Halten der Kammer (1) in bezug auf den Träger (8) durch eine Klammer (55) gebildet sind, die gleichzeitig zwischen zwei Klemmbacken (56, 57) die Kammer (1) und die Stütze (9) festklemmen.

## Claims

1. A fluid infusion device (100) for medical treatment, the device comprising a chamber (1) having at least first and second orifices (2, 3), first means (4) for connecting the first orifice (2) to a source (5) of said medical fluid, a tube (6) of relatively flexible material, second means (7) for connecting the second orifice (3) to said tube, and controllable means (14) for flattening said tube (6) to cause the value of its section (15) to vary in determined manner, a support (8) comprising a base (10), said base having first and second ends (11, 12), said controllable means (14) being suitable for flattening said tube (6) against said base (10), the device being **characterized by** the fact that it further comprises:
fixing means (13) for fixing said chamber (1) relative to the upright (9) of said support (8), said support (8) being constituted by a single upright (9);
said base (10) being secured to said upright via its first end (11) in such a manner that said upright and said base substantially form an L-shape.

2. An infusion device according to claim 1, **characterized by** the fact that the controllable means (14) for flattening said tube (6) against said base (10) comprise a tab (20), means (21) for mounting said tab to pivot on said base, means for exerting a resilient force between said base and said tab tending to increase the value of the angle (22) between said tab (20) and said base (10), and means (23) for controlling a determined reduction in the value of said angle (22) against said resilient force.

3. An infusion device according to claim 2, **characterized by** the fact that the means (21) for monitoring said tab (20) to pivot on said base (10) are constituted by a deformable wall (24) secured to one end (25) of said tab (20) and to the second end (12) of said base (10) in such a manner that said base and said tab are normally substantially parallel facing each other, and being spaced apart by a distance that is substantially equal to the thickness of said tube when it is not flattened.

4. An infusion device according to claim 3, **characterized by** the fact that said tab (20), said deformable wall (24), and said base (10) form a single substantially U-shaped piece, and that the means for exerting a resilient force between said base and said tab are constituted by the fact that said single piece is made of a material that is somewhat resilient.

5. An infusion device according to any one of claims 2 to 4, **characterized by** the fact that the means (23) for controlling variation in the value of said angle (22) between said tab (20) and said base (10) are constituted by a screw (30) having a threaded shank (31) and a head (32) secured to one end (33) of said threaded shank (31), said shank (31) passing through one of the two elements (10) constituted by the tab (20) and the base (10) via a hole (34) of diameter not less than the diameter of said shank (31) and co-operating with a hole (35) formed in the other element (20) and tapped with the same pitch as the pitch of said threaded shank.

6. An infusion device according to claim 4 or claim 5, **characterized by** the fact that said single piece has longitudinal cutouts (40) whose axes are substantially perpendicular to the pivot plane of said tab (20) relative to said base (10).

7. An infusion device according to any one of claims 2 to 6, **characterized by** the fact that it includes at least one groove (41) for receiving said tube (6), said groove (41) being formed in one of the two elements (20) constituted by the tab (20) and the base (10), so as to be perpendicular to the pivot plane of the tab relative to the base, and opening (42) out into the space (43) defined between said two elements (10, 20).

8. An infusion device according to any one of claims 1 to 7, **characterized by** the fact that it includes detector means (50) for detecting the level (59) of said fluid (100) in said chamber (1) when it reaches a reference level.

9. An infusion device according to claim 8, **characterized by** the fact that the detector means (50) comprise at least one sensor (51) suitable for delivering an output signal (52) when said fluid level in said chamber (1) reaches the reference level, said sensor (51) being disposed in a housing (53) formed in said upright (9).

10. An infusion device according to any one of claims 1 to 9, **characterized by** the fact that the means (13) for holding said chamber (1) relative to said support (8) are constituted by a clamp (55) simultaneously clamping said chamber (1) and said upright (9) between two jaws (56, 57).
